# EUROPEAN PATENT SPECIFICATION

(11) **EP 4 451 918 B1**
(45) Date of publication and mention of the grant of the patent: **25.02.2026**
(21) Application number: 22843213.4
(22) Date of filing: 20.12.2022
(51) Int. Cl.: A61K 35/745, A61K 35/747, A61K 39/40, C07K 16/12, A23L 33/135, A23L 33/00, A61P 31/12, A61P 31/16, A61P 37/00

(54) **SECRETORY IGA-BIOTIC COMPLEXES AND USES THEREOF**
SEKRETORISCHE IGA-BIOTISCHE KOMPLEXE UND DEREN VERWENDUNG
COMPLEXES D'IGA-BIOTIQUES SÉCRÉTOIRES ET LEURS UTILISATIONS

(30) Priority: 20.12.2021 EP 21215986
(43) Date of publication of application: 30.10.2024
(60) Divisional application: 26152387.2
(73) Proprietor: Société des Produits Nestlé S.A., 1800 Vevey (CH)
(72) Inventor: NOTI, Mario, 1085 Vulliens (CH); FORBES-BLOM, Elizabeth, 1093 La Conversion (CH); VIDAL, Karine, 1010 Lausanne (CH); BOURDEAU, Tristan, 01170 Echevenex (FR); LUKJANENKO, Laura, 1007 LAUSANNE (CH)
(74) Representative: Loiseau, François Michel
(86) International application number: PCT/EP2022/087070
(87) International publication number: WO 2023/118197

(56) References cited:
- WO-A1-2010/033768
- WO-A1-2020/260502
- WO-A1-2021/181272
- US-A1- 2006 018 890
- US-A1- 2012 014 963
- US-B2- 9 763 465
- SINGH KULJIT ET AL: "Probiotics: A potential immunomodulator in COVID-19 infection management", NUTRITION RESEARCH, vol. 87, March 2021 (2021-03-01), AMSTERDAM, NL, pages 1 - 12, XP055904394, ISSN: 0271-5317, DOI: 10.1016/j.nutres.2020.12.014

## Description

### Field of the invention

The present invention relates to complexes between secretory immunoglobulin A (slgA) and biotics, wherein said biotics are bacteria. Such sIgA-biotic complexes are useful to promote immune defence to prevent or treat viral infections in mammals.

### Background of the invention

A viral infection occurs when the virus proliferates inside the host's cells, therefore utilising the host's resources to promote its own multiplication. Viral infections can also interfere with the normal functioning of the host, leading to more severe infection-related disorders including increased risk of concurrent or secondary bacterial infections and associated antibiotic use as well as long term alterations and subsequent inflammatory diseases later in life.

In particular, severe respiratory syncytial virus (RSV) induced bronchiolitis is a major cause of morbidity and mortality in infants globally (Nair et al, Lancet, 2010;375;9725;2545-1555). Severe bronchiolitis is associated with bacterial co-infections (Thorburn K et al, Thorax 2006;61(7):611-615) or secondary infections (Sande CJ et al, Nature Communications 2019 10, 2218) resulting in increased antibiotic use. Further, severe viral airway infections in early life represent and independent risk factor for subsequent development of pulmonary diseases such as allergic airway disease (e.g. asthma, Feldman et al, Am J Respir Crit Care Med, 2015;191;34-44) and chronic obstructive pulmonary disease (Savran O et al. Int J Chron Obstruct Pulmon Dis. 2018; 13: 683-693) in later life. Breastfeeding is a recognised factor that reduces the severity of viral infections in infants. Secretory IgA (slgA) is a notable component of breast milk as infants do not produce slgA during the early stages of life and as such they rely on passive transfer of slgA and slgA-bacteria complexes contained in mammalian milk (Dzidic M et al. Pediatr Allergy Immunol, 2020; 31(3):250-257). sIgA is known to drive the establishment and maintenance of the infant gut microbiome through specific and unspecific mechanisms. Besides specific binding of sIgA to pathogens (Fab mediated) to mediate immune exclusion and pathogen neutralization, sIgA can also bind to commensal bacteria and probiotics (non-specific binding). Many of these IgA-commensal interactions are glycan mediated, either from bacterial surface glycan structures or through N-linked glycans glycosylation of the secretory component. Such slgA-bacteria complexes can be either provided through milk or are formed in the gastrointestinal tract where luminal sIgA binds to commensal bacteria. sIgA-bacteria complexes are found in adult and infant gastrointestinal microbiota as well as in milk. sIgA-bacteria complexes differ in composition that is influenced by age and diet of the host and members of the same bacteria species can be found either complexed - or non-complexed to slgA (Ding M et al, Cell 2021 29; 8: 725-735). sIgA-bacteria may translocate from the maternal gastrointestinal tract to milk via the entero-mammary link (Salvamani S et al, Appl. Sci. 2021, 11, 7247) and modulate intestinal immunity via unique modes (Ding M et al, Cell 2021 29; 8: 725-735).

It is known that probiotics, in particular from the Lactobacillus and Bifidobacterium genus, support protection against respiratory tract infections. The role of probiotics in viral respiratory tract infections was reviewed by Lehtoranta and co-workers (Lehtoranta et al, Eur J Clin Microbiol Infect Dis, 2014;33; 1289-1302). Importantly, association of sIgA with probiotics has been demonstrated to allow crosstalk between the probiotic and the host (see Mathias et al, JBC, 2010;285(44);33906-33913; Rol et al, JBC, 2012;287(47);40074-40082; and Mathias et al, Gut Microb, 2014;5(6);688-695).

While some infant formulas contain probiotics to promote immune maturation, current infant formulas lack functional sIgA since sIgA is degraded through the extensive heat treatment required to ensure microbiological safety. However, it was recently demonstrated in WO 2020/260502 A1 to preserve active sIgA in bovine milk and to complex slgA with spray dried probiotics. Compositions comprising slgA complexed with probiotics for the purpose of treating/preventing inflammation and for the purpose of treating/preventing non-viral infections are described in WO 2009/156301 A1 and WO 2009/156307 A1, respectively. sIgA-probiotic complexes, wherein the probiotic has been "HMO-activated", for combatting entero-pathogens are disclosed in WO 2021/062049.

Although sIgA complexed with bacteria have been described to be useful to prevent or treat inflammation, bacterial infections or to reduce infections by entero-pathogens, there is no indication in the prior art that sIgA complexed with beneficial bacteria, either probiotic or postbiotic, can help to prevent or treat viral infections. Additionally, there are nowadays limited means to prevent or treat viral infections. There are limited numbers of effective antiviral drugs, for example drugs used to treat HIV and influenza, and the primary method to control viral disease is vaccination which is intended to prevent outbreaks by building immunity to a virus or a family of viruses.

It is therefore an object of the present invention to provide a composition to prevent and/or treat viral infections, and more specifically to prevent or treat viral infections of the respiratory tract.

### Summary of the invention

Using a mouse model of viral bronchiolitis and early life nutritional supplementation, slgA-biotic complexes optimized immune defence against infection as compared to the single ingredients. Specifically, nutritional supplementation with slgA-biotic complexes promoted a significant expansion of plasmacytoid dendritic cells (pDCs) and regulatory T cells (Tregs) in the airways (distant mucosal site) of animals infected with a respiratory virus. The demonstrated induction of a pDC-Treg response via the gut-lung axis was paralleled by optimal immune protection from viral infection. Thus, a composition comprising slgA-biotic complexes will be useful to promote optimal immunity, defined as a context-specific, dose-duration regulated immune defence against an external challenge. Such responses are tightly regulated to mount efficient strength at peak of the challenge while rapidly returning to a homeostatic state upon clearance of such threat (resilience) to prevent harmful responses against the bodies' own tissue while allowing for appropriate tissue remodelling and regain of tissue/organ function.

These findings suggest that early life nutritional supplementation with slgA-biotic complexes promote optimal immunity to prevent and/or treat bronchiolitis and thus may reduce the risk of concurrent or secondary bacterial infections and associated use of antibiotics, and/or limit long-term complications later in life (e.g. asthma) associated with viral respiratory infections in early life.

The present invention relates to a composition comprising secretory immunoglobulin A-biotic (sIgA-biotic) complexes, wherein said biotic is a bacterium, wherein said bacterium is a Bifidobacterium or a Lactobacillus, for use in prevention and/or treatment of a viral infection in a mammal as defined in the appended claims.

### Description of the figures

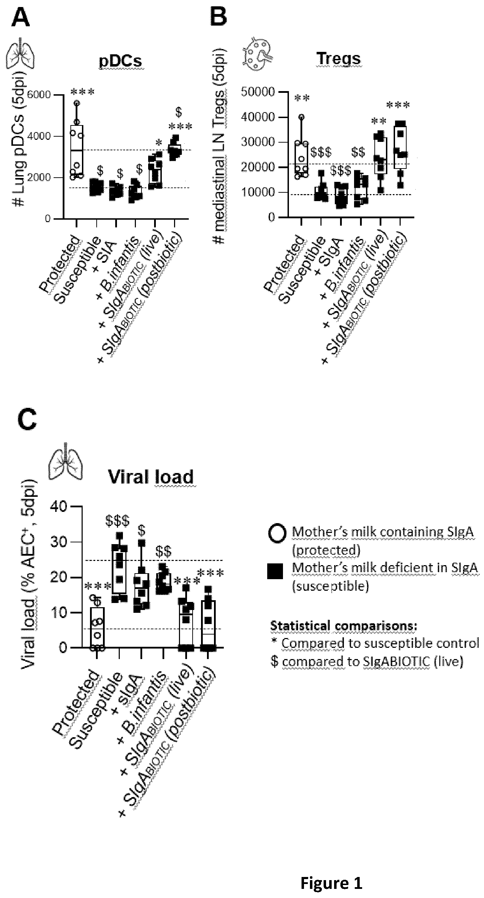

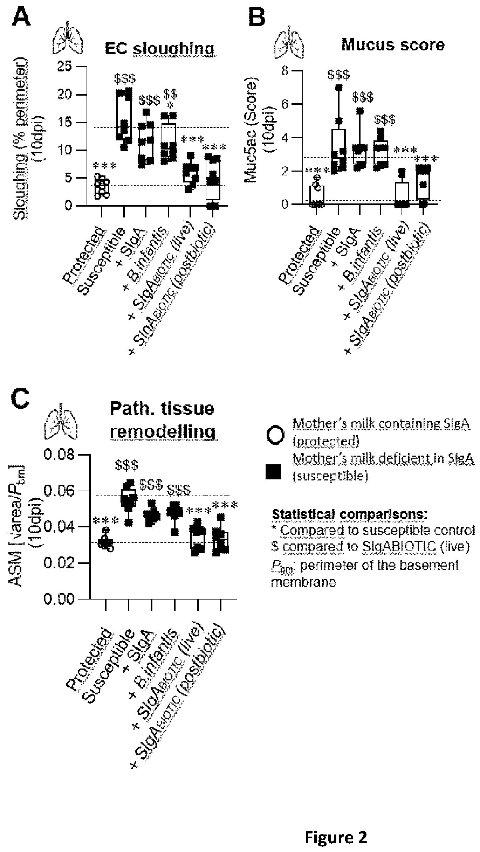

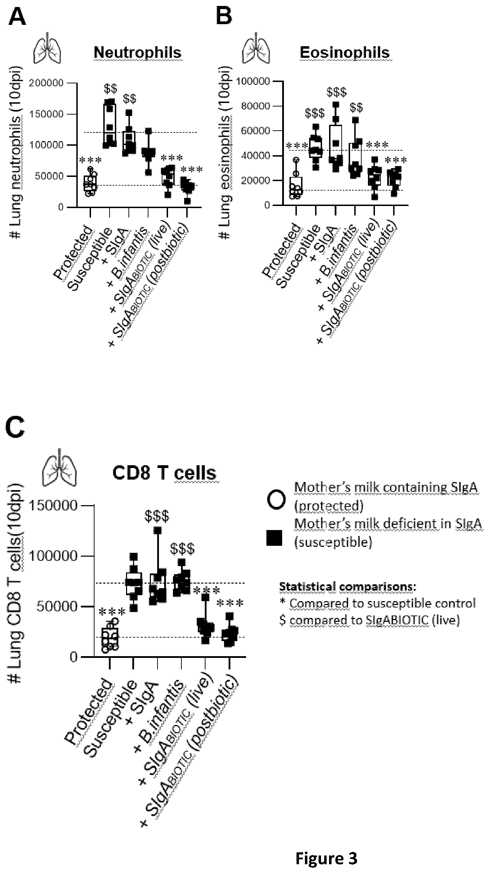

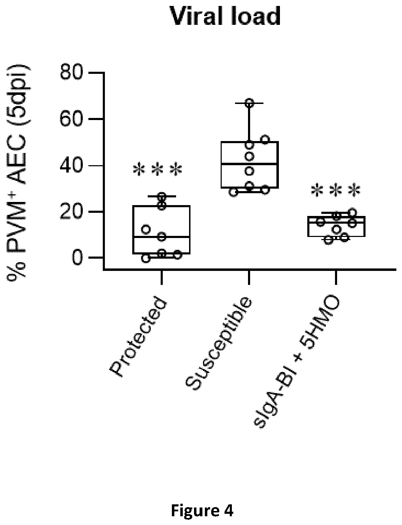

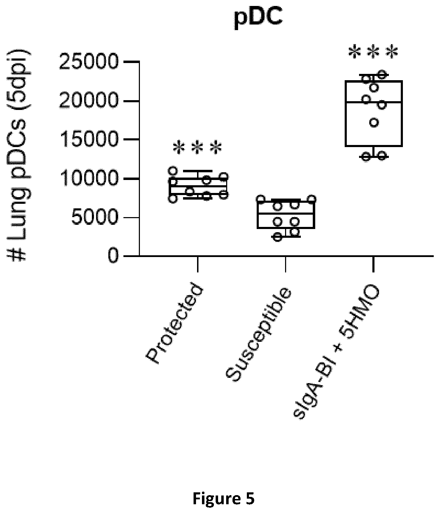

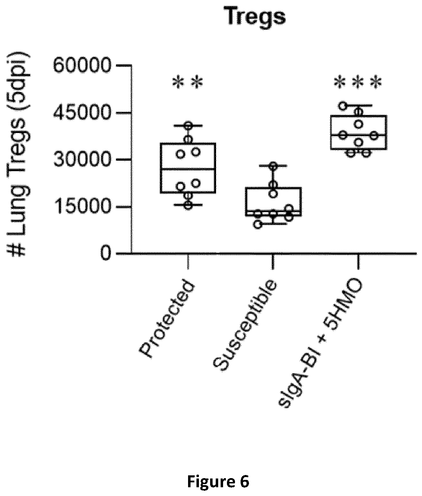

**Figure 1** shows the (A) numbers of plasmacytoid dendritic cells (pDCs) in lung tissues of mice infected with Pneumonia Virus of Mice (PVM) 5 days post-infection (dpi); (B) numbers of regulatory T cells (Tregs) in mediastinal lymph nodes (LN) of PVM infected animals at 5dpi; (C) viral load assessed by percentage of PVM positive airway epithelial cells (AEC) at 5 dpi. N=8 per group. Statistical analysis was performed by two-way analysis of variance ANOVA. *compared to susceptible controls. $ compared to sIgABIOTIC (live). */$ p < 0.01; **/$$ p < 0.05; ***/$$$ p < 0.001.
**Figure 2** shows (A) epithelial cell (EC) sloughing, (B) mucus score as assessed by Muc5ac lung tissue staining and (C) pathological tissue remodeling assessed by alpha-smooth muscle (ASM) actin staining in lung tissue of PVM infected mice at 10dpi. P_{bm}: perimeter per basement membrane. N=8 per group. Statistical analysis was performed by two-way ANOVA. * Compared to susceptible controls. $ compared to sIgABIOTIC (live). */$ p < 0.01; **/$$ p < 0.05;***/$$$ p < 0.001
**Figure 3** shows flow cytometric quantification of (A) lung tissue neutrophil, (B) eosinophil and (C) CD8 T cell numbers at 10dpi. N=8 per group. Statistical analysis was performed by two-way ANOVA.. * compared to susceptible controls. $ compared to sIgABIOTIC (live). $$ p < 0.05; ***/$$$ p < 0.001.
**Figure 4** shows viral load assessed by percentage of PVM positive airway epithelial cells (AEC) at 5 dpi in lungs of PVM infected protected-, susceptible- and susceptible mice supplemented with slgABIOTIC (live) in the presence of 5HMOs. Data are analyzed by two-way analysis of variance (ANOVA) and compared to susceptible groups. **p ≤ 0.01; ***p ≤ 0.001.
**Figure 5** shows the number of plasmacytoid dendritic cells (pDC) in lung of PVM infected protected, susceptible and susceptible mice supplemented with slgABIOTIC (live) in the presence of 5HMOs. Data are analyzed by two-way analysis of variance (ANOVA) and compared to susceptible groups. **p ≤ 0.01; ***p ≤ 0.001.
**Figure 6** shows the number of regulatory T cells (Tregs) in lung of PVM infected protected, susceptible and susceptible mice supplemented with slgABIOTIC(live) in the presence of 5HMOs. Data are analyzed by two-way analysis of variance (ANOVA) and compared to susceptible groups. **p ≤ 0.01; ***p ≤ 0.001.

### Detailed description of the invention

The present invention relates to a composition comprising secretory immunoglobulin A-biotic (sIgA-biotic) complexes, wherein said biotic is a bacterium, wherein said bacterium is a Bifidobacterium or a Lactobacillus, for use in the prevention and/ or treatment of viral infection in a mammal, as defined in the appended claims.

In one embodiment the composition for use of the present invention comprises sIgA complexed with at least one probiotic.

In one embodiment, the composition for use of the present invention comprises slgA-postbiotic complexes.

The composition for use of the present invention may also contain slgA-biotic complexes along with biotics that are not complexed with sIgA.

Immunoglobulins (also called antibodies) are often glycoproteins, which specifically recognise antigens. In mammals, at least five immunoglobulin classes are described, including IgG, IgM, IgA, IgD and IgE, all of which differ in their abundance and function. Secretory IgA (SIgA), which is the predominant immunoglobulin in intestinal mucosal secretions, was found to be in particular effective for the purpose of the present invention. Within the context of the present invention, the expressions "secretory IgA", "sIgA" and "SIgA" can be used interchangeably.

The sIgA may be from a milk source or may be recombinant or synthetic or cell-protein expression system. The source of SlgA is typically of milk or a milk fraction. In some embodiments, the milk is cow's milk, buffalo milk, sheep's milk, goat's milk, human milk, horse milk or camel milk. In preferred embodiments, the milk is cow's milk. In a preferred embodiment, the milk is skimmed milk. In some embodiments, the milk is colostrum. In some embodiments, the milk fraction is whey. In an embodiment, the milk used as the source of slgA is concentrated milk. In a preferred embodiment, the milk is first skimmed by centrifugation and then concentrated by reverse osmosis.

Secretory IgA can be associated with probiotic and form complexes as described for example in

WO 2020/260502 or US 2012/014963 A1.

The first interaction of probiotics with the host occurs at the level of the gut mucosa. Among the key criteria for the selection of a probiotic micro-organism is its capacity to adhere to intestinal mucosa. This adhesion seems to be required to block pathogen entry and to contribute to modulate protective immune functions, for example.

One of the most characteristic features of the mucosal immune system in most mammals is the dominant presence of secretory antibodies, particularly secretory IgA (SIgA), an antibody class unique to mucosae. Biosynthesis of polymeric IgA takes place in the mucosal lamina propria, and its transport across the epithelium lining the mucosal surfaces is ensured by the polymeric Ig receptor (plgR) expressed by crypt and columnar epithelial cells.

In secretions, a significant portion of the plgR termed the secretory component (SC) remains associated with polymeric IgA, releasing SIgA.

The release of SIgA into the lumen is dependent on the production of SC, whose expression is up regulated after birth. pIgR appears to be critical to the stability and mucosal anchoring of the antibody (Phalipon et al. 2002 Secretory component: A new role in secretory IgA-mediated immune exclusion in vivo. Immunity;17:107-115).

Neonates are not yet able to produce endogenous SIgA in quantities sufficient to provide passive immunity. In the first days of life, neonates rely on passive immunity provided from their mothers via IgG transferred through the placenta and on an exogenous supply of SIgA abundantly found in breast milk.

Together, this confers passive immunity essential to the protection of the host during the phase of shaping and maturation of immune system.

Hence the composition for use of the present invention will be in particular beneficial for newborns and infants (up to 2 years old), since they do not produce SIgA in sufficient amounts but rely on external supply.

The inventors show that a composition comprising slgA-biotic is particularly efficient to prevent and/or treat viral infections. Surprisingly, the inventors show that a composition comprising sIgA complexed with postbiotics is at least equally efficient to prevent and/or treat viral infection as compared to a composition comprising sIgA complexed with probiotics. The inventors presently believe that it is this association of SIgA with biotic, that potentiates the interaction with the host, so that the resulting health benefits for the host are improved.

The present inventors have identified secretory IgA antibodies capable of associating with biotic.

It is widely accepted that while some microorganisms, and in particular bacteria, can be harmful and cause disease, other play beneficial role in human health. Beneficial bacteria are broadly designated under the term of probiotics.

"Probiotics" are defined as "live microorganisms that, when administered in adequate amounts, confer a health benefit on the host" (Hill C, et al. 2014 "Expert consensus document. The International Scientific Association for Probiotics and Prebiotics consensus statement on the scope and appropriate use of the term probiotic." Nat. Rev. Gastroenterol. Hepatol.; 11:506-514). Probiotics are by definition alive and required to have an efficacious amount of viable bacteria at the time of administration to the host, but most probiotic preparations, especially at the end of shelf life, will also include potentially large numbers of dead and injured microorganisms (Fiore W. et al. 2020 Microorganisms; 8:1177; Holmes E, et al. 2012 Sci. Transl. Med.; 4:137rv136).

Recent interest for the health benefits of non-viable microorganisms, their components and their end-products resulted in the concept of postbiotics. "Postbiotics" are defined as a "preparation of inanimate microorganisms and/or their components that confer a health benefit on the host" (Salminen S et al., 2021 The International Scientific Association of Probiotics and Prebiotics (ISAPP) consensus statement on the definition and scope of postbiotics. Nat. Rev. Gastroenterol. Hepatol.; 18: 649-667).

Postbiotics are deliberately inactivated microbial cells with or without metabolites or cell components that contribute to demonstrated health benefits. A postbiotic does not have to be derived from a probiotic for the inactivated version to be accepted as a postbiotic (Salminen S et al. 2021 "The International Scientific Association of Probiotics and Prebiotics (ISAPP) consensus statement on the definition and scope of postbiotics." Nat. Rev. Gastroenterol. Hepatol.; 18: 649-667).

Postbiotics can be intact microorganisms, microbial cell components, including pili, cell wall components or other structures. The presence of microbial metabolites or end products of growth on the specified matrix produced during growth and/or fermentation is also anticipated in some postbiotic preparations, although the definition would not include substantially purified metabolites in the absence of cellular biomass (Salminen S et al., 2021 The International Scientific Association of Probiotics and Prebiotics (ISAPP) consensus statement on the definition and scope of postbiotics. Nat. Rev. Gastroenterol. Hepatol. 18: 649-667).

Inactivate microorganisms can be obtained by thermal processing, e.g. pasteurization, tyndallization, and autoclaving, or non-thermal inactivation techniques such as electric field, ultrasonication, high pressure, X-rays, ionizing radiation, high-voltage electrical discharge, pulsed light, magnetic field heating, moderate magnetic field and plasma technology.

A microbial strain or consortium does not have to qualify as a probiotic (while living) for the inactivated version to be accepted as a postbiotic (Salminen S et al., 2021 "The International Scientific Association of Probiotics and Prebiotics (ISAPP) consensus statement on the definition and scope of postbiotics." Nat. Rev. Gastroenterol. Hepatol.; 18: 649-667). Specific strains of *Akkermansia muciniphila, Faecalibacterium prousnitzii., Bacteroides xylanisolvens, Bacteroides uniformis, Eubacterium hallii, Clostridium cluster* IV and XIVa, *Apilactobacillus kunkeei* and the fungus *Saccharomyces boulardii* would fit the definition of postbiotic should a health benefit be demonstrated.

The probiotic and/or postbiotic microorganis used in accordance with the present invention are selected from the group consisting of Bifidobacterium or Lactobacillus, in particular selected from the group consisting of *Bifidobacterium longum* subspecies *longum, Bifidobacterium longum* subsp. *infantis, Bifidobacterium breve, Bifidobacterium bifidum, Bifidobacterium adolescentis, Bifidobacterium animalis, Bifidobacterium longum, Bifidobacterium lactis, Lactobacillus acidophilus, Lactobacillus rhamnosus, Lactobacillus paracasei, Lactobacillus johnsonii, Lactobacillus plantarum, Lactobacillus* salivarius and *Lactobacillus reuteri* or mixtures thereof, preferably selected from the group consisting of *Lactobacillus johnsonii* (NCC533; CNCM 1-*1225), Bifidobacterium lactis* BI07, *Bifidobacterium lactis* BL04, *Bifidobacterium lactis* HN019, *Bifidobacterium longum* subsp. *infantis* ATCC 17930 (LMG 11588; NCC 3089) and *Bifidobacterium infantis* ATCC 15697 (NCC 3078), *Bifidobacterium juvenis* (NCC 5000-5004), *Bifidobacterium longum* (NCC490; CNCM I- 2170), *Bifidobacterium longum* (NCC2705; CNCM 1-2618), *Bifidobacterium lactis* (NCC2818; CNCM I-3446), *Lactobacillus paracasei* (NCC2461; CNCM 1-2116), *Lactobacillus rhamnosus GG* (ATCC 53103), *Lactobacillus rhamnosus* (NCC4007; CGMCC 1.3724), and mixtures thereof.

The sIgA-biotic complexes used in this invention are preferably preformed prior to consumption, wherein SIgA and at least one bacteria, probiotic and/or postbiotic, are at least partially associated.

The SIgA and the biotics are present in a stoichiometric ratio of at least 10:1, preferably at least 100:1, most preferably at least 2000:1 to 100000:1. The upper limit of sIgA saturation is determined by the surface of the microorganisms and/or components, and by the number of available binding sites for SIgA.

The composition comprises between 0,0001 and 100 mg SIgA per daily dose. The composition comprises between 10² and 10¹² cells of biotics, probiotics or postbiotics, per daily dose. Wherein at least 50%, preferably 70%, most preferably more than 90% of the biotics are associated with at least one SIgA molecule as complexes. In one embodiment, at least 50%, preferably 70%, most preferably more than 90% of the probiotics are associated with at least one SIgA molecule as complexes. In another embodiment, at least 50%, preferably 70%, most preferably more than 90% of the postbiotics are associated with at least one SIgA molecule as complexes.

The composition of the present invention may also contain prebiotics. The addition of prebiotics is beneficial as it can, when combined with probiotics deliver synergistic effects in terms of the health benefits. A composition comprising a combination of prebiotics and probiotics is commonly known as a synbiotic composition.

"Prebiotic" is defined as being a "substrate that is selectively utilized by host microorganisms conferring a health benefit" (Gibson GR, et al., 2017 "Expert consensus document: The International Scientific Association for Probiotics and Prebiotics (ISAPP) consensus statement on the definition and scope of prebiotics." Nat. Rev. Gastroenterol. Hepatol.;14:491-502).

They are not broken down in the stomach and/or upper intestine or absorbed in the gastrointestinal tract of the person ingesting them, but they are fermented by the gastrointestinal microflora and/or by probiotics. Prebiotics are for example defined by Glenn R. Gibson and Marcel B. Roberfroid, Dietary Modulation of the Human Colonic Microbiota: Introducing the Concept of Prebiotics. J. Nutr.; 1995 125: 1401 -1412.

The prebiotics that may be used in accordance with the present inventions are not particularly limited and include all food substances that promote the growth of beneficial bacteria such as bifidobacteria or lactobacilli, and/or probiotics. Preferably, they may be selected from the group consisting of oligosaccharides, optionally containing fructose, galactose, mannose; dietary fibers, in particular soluble fibers, soy fibers; inulin; or mixtures thereof. Preferred prebiotics are fructo-oligosaccharides (FOS), galacto-oligosaccharides (GOS), isomalto- oligosaccharides, xylo-oligosaccharides, oligosaccharides of soy, glycosylsucrose (GS), lactosucrose (LS), lactulose (LA), palatinose-oligosaccharides (PAO), malto- oligosaccharides, pectins and/or hydrolysates thereof. The composition of the invention is effective for use in therapy.

The composition of the present invention may include one or more human milk oligosaccharides (HMO or HMOs). In some other embodiments, the composition of the present invention may include Bovine Milk one or more oligosaccharides (BMOs). HMOs play an essential role in the early development of infants and young children, such as the maturation of the immune system. Each individual oligosaccharide is based on a combination of glucose, galactose, sialic acid (N-acetylneuraminic acid), fucose and/or N-acetylglucosamine with many and varied linkages between them, thus accounting for the enormous number of different oligosaccharides in human milk (over 130 structures). Almost all HMOs have a lactose moiety at their reducing end while sialic acid and/or fucose /when present) occupy terminal positions at the non-reducing ends. The HMOs can be acidic (e.g charged sialic acid containing oligosaccharides) or neutral (e.g., fucosylated oligosaccharide).

In some embodiments, the composition according to the present invention may comprise HMOs in a total amount of from 1000 to 10'000 mg/L, preferably from 1500 to 8000 mg/L, more preferably from 2000 to 5000 mg/L, even more preferably from 3000 to 4000 mg/L, even more preferably from 3590 to 3673 mg/L, and most preferably in an amount of 3632 mg/L of composition. In some embodiments, the compositions described herein include HMOs in an amount of from 1500 to 3500 mg/L, preferably from 2000 to 3000 mg/L, more preferably from 2558 to 2602 mg/L, and most preferably in an amount of 2580 mg/L of composition. In some embodiments, the compositions described herein include HMOs in an amount of from 500 to 2500 mg/L, preferably from 1500 to 2000 mg/L, more preferably from 1863 to 1902 mg/L, and most preferably in an amount of 1883 mg/L of composition.

In some embodiments the oligosaccharide(s) is or includes one or more fucosylated oligosaccharides. A "fucosylated oligosaccharide" is an oligosaccharide having a fucose residue. It has a neutral nature. Some examples are 2-FL (2'-fucosyllactose), 3-FL (3-fucosyllactose), difucosyllactose, lacto-N-fucopentaose (e.g., lacto-N-fucopentaose I, lacto-N-fucopentaose II, lacto-N-fucopentaose III, lacto-N-fucopentaose V), lacto-N-fucohexaose, lacto-N-difucohexaose I, fucosyllacto-N-hexaose, fucosyllacto-N-neohexaose, difucosyllacto-N-hexaose I, difucosyllacto-N-neohexaose II and any combination thereof.

The expressions "fucosylated oligosaccharides comprising a 2'-fucosyl-epitope" and "2-fucosylated oligosaccharides" encompass fucosylated oligosaccharides with a certain homology of form since they contain a 2'-fucosyl-epitope, therefore a certain homology of function can be expected.

In some particular embodiments the fucosylated oligosaccharide comprises a 2'-fucosyl-epitope. It can be for example selected from the list comprising 2'-fucosyllactose, difucosyllactose, lacto-N-fucopentaose, lacto-N-fucohexaose, lacto-N-difucohexaose, fucosyllacto-N-hexaose, fucosyllacto-N-neohexaose, difucosyllacto-N-hexaose difuco-lacto-N-neohexaose, difucosyllacto-N-neohexaose, fucosyl-para-Lacto-N-hexaose and any combination thereof.

In some embodiments, the synthetic formulation includes a 2'-fucosyllactose (or 2FL, or 2'FL, or 2-FL or 2'-FL). In a particular embodiment, there is no other type of fucosylated oligosaccharide than 2'-fucosyllactose, i.e., the nutritional composition of the invention comprises only 2'-fucosyllactose as fucosylated oligosaccharide.

The fucosylated oligosaccharide(s) can be present in the nutritional composition according to the present invention in a total amount of 0.2-3 g/L, for example 0.5-2 g/L or 0.75-1.65 g/L of the composition. In some embodiments, the fucosylated oligosaccharide(s) may be in a total amount of 0.8-1.5 g/L of the composition, such as 0.85-1.3 g/L or 0.9-1.25 g/L or 0.9-1.1 g/L or 1-1.25 g/L or 1.05-1.25 g/L of the composition. In a particular embodiment, the fucosylated oligosaccharide(s) is/are in a total amount of 1 g/L of the composition. In another particular embodiment, the fucosylated oligosaccharide(s) is/are in a total amount of 1.24 g/L of the composition. The fucosylated oligosaccharide(s) can be present in the nutritional composition in a total amount of 0.13-2.1 g/100 g, for example 0.34-1.4 g/100 g or 0.52-1.15 g/100 g of composition on a dry weight basis. The fucosylated oligosaccharide(s) may be in a total amount of 0.55-1.05 g/100 g of the composition, such as 0.59-0.9 g/100 g, or 0.62-0.87 g/100 g or 0.62-0.77 g/100 g or 0.69-0.87 g/100 g or 0.73-0.87 g/100 g of the composition. In a particular embodiment, the fucosylated oligosaccharide(s) is/are in a total amount of 0.69 g/100 g of the composition. In another particular embodiment, the fucosylated oligosaccharide(s) is/are in a total amount of 0.86 g/100 g of the composition.

In some embodiments the oligosaccharide(s) is or includes one or more N-acetylated oligosaccharides. The expression "N-acetylated oligosaccharide(s)" encompasses both "N-acetyl-lactosamine" and "oligosaccharide(s) containing N-acetyl-lactosamine". They are neutral oligosaccharides having an N-acetyl-lactosamine residue. Suitable examples are LNT (lacto-N-tetraose), para-lacto-N-neohexaose (para-LNnH), LNnT (lacto-N-neotetraose) or any combination thereof. Other examples are lacto-N-hexaose, lacto-N-neohexaose, para-lacto-N-hexaose, para-lacto-N-neohexaose, lacto-N-octaose, lacto-N-neooctaose, iso-lacto-N-octaose, para-lacto-N-octaose and lacto-N-decaose.

In some specific embodiments, the synthetic formulation can include at least one the N-acetylated oligosaccharide. There can be one or several types of N-acetylated oligosaccharide. The N-acetylated oligosaccharide(s) can be for example lacto-N-tetraose (LNT), lacto-N-neotetraose (LNnT) or any combination thereof. In some particular embodiments the N-acetylated oligosaccharide is lacto-N-neotetraose (LNnT), para-lacto-N-neohexaose (para-LNnH) or any combination thereof. In some particular embodiments the N-acetylated oligosaccharide is LNnT. In some particular embodiments the N-acetylated oligosaccharide is LNT. In some other particular embodiments, the N-acetylated oligosaccharide is a mixture of LNT and LNnT. In some particular embodiments the composition comprises both LNT and LNnT in a ratio LNT:LNnT between 5:1 and 1:2, or from 2:1 to 1:1, or from 2:1.2 to 2:1.6.

The N-acetylated oligosaccharide(s) can be present in the nutritional composition according to the present invention in a total amount of 0.1-2 g/L, for example 0.3-1 g/L or 0.45-0.85 g/L of the composition.

In some embodiments, the N-acetylated oligosaccharide(s) may be in a total amount of 0.5-0.8 g/L of the composition, such as 0.5-0.75 g/L or 0.5-0.7 g/L of the composition. In a particular embodiment, the N-acetylated oligosaccharide(s) is/are in a total amount of 0.5 g/L of the composition. In another particular embodiment, the N-acetylated oligosaccharide(s) is/are in a total amount of 0.63 g/L of the composition.

The N-acetylated oligosaccharide(s) can be present in the nutritional composition in a total amount of 0.06-1.4 g/100 g, for example 0.2-0.7 g/100 g or 0.31-0.59 g/100 g of composition on a dry weight basis, The N-acetylated oligosaccharide(s) may be in a total amount of 0.35-0.56 g/100 g of composition, such as 0.35-0.52 g/100 g or 0.35-0.49 g/100 g. In a particular embodiment, the N-acetylated oligosaccharide(s) is/are in a total amount of 0.35 g/100 g of the composition. In another particular embodiment, the N-acetylated oligosaccharide(s) is/are in a total amount of 0.44 g/100 g of the composition.

In some other particular embodiments, the synthetic formulation does not comprise any N-acetylated oligosaccharides

In some embodiments, the synthetic formulation comprises both 2'-fucosyllactose (2-FL) and lacto-N-neotetraose (LNnT). In another specific embodiment, the nutritional composition of the present invention comprises an oligosaccharide mixture that consists of 2'-fucosyllactose (2-FL) and lacto-N-neotetraose (LNnT). In other words, the nutritional composition of the invention comprises only 2'-fucosyllactose (2-FL) as fucosylated oligosaccharide and only lacto-N-neotetraose (LNnT) as N-acetylated oligosaccharide.

The synthetic formulation can include fucosylated oligosaccharide(s) and N-acetylated oligosaccharide(s) in a ratio fucosylated oligosaccharide(s): the N-acetylated oligosaccharide(s) of from 2:0.065 to 2:20 such as from 2:0.3 to 2:4, for example 2:0.54 to 2:2.26, or 2:0.76-2:1.8 or 2:0.8-2:1.4. In a particularly advantageous embodiment, this ratio is 2:1 or around 2:1.

In some embodiments the oligosaccharide(s) is or includes one or more sialylated oligosaccharides.

A "sialylated oligosaccharide" is a charged sialic acid containing oligosaccharide, i.e., an oligosaccharide having a sialic acid residue. It has an acidic nature. Some examples are 3-SL (3' sialyllactose) and 6-SL (6' sialyllactose). The sialylated oligosaccharide(s) can be selected from the group comprising 3' sialyllactose (3-SL), 6' sialyllactose (6-SL), and any combination thereof. In some embodiments of the invention the composition comprises 3-SL and 6-SL. In some particular embodiments the ratio between 3'-sialyllactose (3-SL) and 6'-sialyllactose (6-SL) can be in the range between 5:1 and 1:10, or from 3:1 and 1:1, or from 1:1 to 1:10. In some specific embodiments the sialylated oligosaccharide of the composition is 6' sialyllactose (6-SL).

In particular examples, the synthetic formulation includes from 0.05 to 5 g/L of sialylated oligosaccharide(s), or from 0.1 to 4 g/L, or from 0.3 to 2 g/L, or from 0.4 to 1.5 g/L, or from 0.4 to 1 g/L, for example 0.5 or 0.9 g/L of sialylated oligosaccharide(s). In some particular embodiments, the synthetic formulation includes from 0.8 to 1.7 g/l of sialylated oligosaccharide(s).

The synthetic formulation includes can contain from 0.03 to 3.5 g of sialylated oligosaccharide(s) per 100 g of composition on a dry weight basis, e.g., from 0.1 to 2 g or from 0.2 to 1 g or from 0.3 to 0.6 g of sialylated oligosaccharide(s) per 100 g of composition on a dry weight basis.

The synthetic formulation can include sialylated oligosaccharide(s) in an amount of below 0.1 g/100 g of composition on a dry weight basis.

In some particular embodiments, the synthetic formulation does not contain any sialylated oligosaccharide(s).

In some other particular embodiments of the present invention, the nutritional composition does not contain any galacto-oligosaccharides (GOS).

In some embodiments, the synthetic formulation optionally also includes at least one precursor of oligosaccharide. There can be one or several precursor(s) of oligosaccharide. For example, the precursor of human milk oligosaccharide is sialic acid, fucose or a mixture thereof. In some particular embodiments the composition comprises sialic acid.

In particular examples the composition comprises from 0 to 3 g/L of precursor(s) of oligosaccharide, or from 0 to 2 g/L, or from 0 to 1 g/L, or from 0 to 0.7 g/L, or from 0 to 0.5 g/L or from 0 to 0.3 g/L, or from 0 to 0.2 g/L of precursor(s) of oligosaccharide.

The composition according to the invention can contain from 0 to 2.1 g of precursor(s) of oligosaccharide per 100 g of composition on a dry weight basis, e.g., from 0 to 1.5 g or from 0 to 0.8 g or from 0 to 0.15 g of precursor(s) of oligosaccharide per 100 g of composition on a dry weight basis.

The composition is, in particular effective for use in the treatment and/or prevention of a viral infection in a mammal, such as a human.

Accordingly, the composition is also effective for use in reducing the risk of contracting a viral infection in a mammal and/or for use in reducing the symptoms associated with a viral infection in a mammal, such as a human.

The viral infection is a viral respiratory tract infection. The viral respiratory tract infection may be a viral infection in the upper respiratory tract or in the lower respiratory tract. In a typical embodiment of the invention, the viral respiratory tract infection is caused by respiratory syncytial virus (RSV).

The disease associated with the viral infection will typically be common cold, influenza (flu), bronchitis, bronchiolitis, pneumonia, sore throat (pharyngitis), sinusitis, non-allergic rhinitis, severe acute respiratory syndrome (SARS), viral croup, otitis media, meningitis or diarrhoea. Typically, when the viral infection is in the respiratory tract, the disease associated with the respiratory tract infection is common cold, influenza (flu), bronchitis, bronchiolitis, pneumonia, sore throat (pharyngitis), sinusitis, non-allergic rhinitis, severe acute respiratory syndrome (SARS), viral croup or otitis media. Most often, the disease associated with the viral respiratory tract infection is common cold, influenza (flu), bronchitis, bronchiolitis or pneumonia.

Accordingly, in a preferred embodiment of the invention the composition is for use in treating and/or preventing a disease associated with a viral respiratory tract infection selected from the group consisting of common cold, influenza (flu), bronchitis, bronchiolitis and pneumonia. In a more preferred embodiment, the disease associated with the respiratory tract infection is selected from the group consisting of bronchiolitis and pneumonia, in particular bronchiolitis and pneumonia caused by RSV. In an even more preferred embodiment, the disease associated with the respiratory tract infection is bronchiolitis, in particular bronchiolitis caused by RSV.

The symptoms most often associated with the viral infection, and which may be reduced by the composition, are irritation in the lungs, congestion in the lungs, excessive mucus production, fever, cough, wheezing, breathlessness, abdominal cramps, diarrhoea or vomiting.

The above-mentioned infections may be caused by a variety of different viruses, including respiratory syncytial virus (RSV), parainfluenza virus (PIV), influenza virus such as influenza virus A (IVA) and/or influenza virus B (IVB), rhinovirus (RV), adenovirus (ADV), metapneumovirus (MPV), bocavirus (BoV), coronavirus (CoV), myxovirus, herpesvirus, enterovirus (EV), parachovirus (PeV) or a combination thereof.

The composition is particularly effective in treating, preventing, reducing the risk of contracting and/or reducing the symptoms of a viral infections caused by respiratory syncytial virus (RSV). Thus, composition of the invention is particularly preferred for use in treating, preventing, reducing the risk of contracting and/or reducing the symptoms of bronchiolitis caused by respiratory syncytial virus (RSV) or pneumonia caused by respiratory syncytial virus (RSV).

Since respiratory viral infections are also a major determinant for development of pulmonary diseases, such as chronic obstructive pulmonary disease (Savran et al, Int J Chron Obstruct, 2015;191;34-44), later in life, the composition of the invention is also suitable for use in preventing or reducing the risk of developing pulmonary diseases, in particular chronic obstructive pulmonary disease, in a mammal, such as a human.

Since viral infections, in particular infection with RSV, is often associated with bacterial co-infection (Thorburn et al, Thorax, 2006;61(7);611-615) or secondary infection (Sande et al, Nature Communications, 2019;10;2218), including antibiotic use, the composition of the invention is also effective for use in preventing or reducing the risk of a bacterial co-infection and/or a bacterial secondary infection associated with respiratory viral infection in a mammal, in particular a human. Pathogenic bacteria typically involved in co-infections or secondary infections include *Staphylococcus aureus, Streptococcus pneumoniae* and/or *Haemophilus influenza.*

The mammal to be treated is preferably a human being, but the mammal may also be non-human mammal, such as a non-human mammal selected from the group consisting of pig, cow, horse, dog, cat, goat, sheep and rabbit.

The composition is useful for treating and/or preventing respiratory tract infection in a human of any age. Thus, the human to be treated with the composition may be selected from the group consisting of 0 to <1 year (infants), 1 to <3 years (young children), 3 to <5 years (pre-schoolers), 5 to <13 years (grade-schoolers), 13 to <20 years (teenagers), 20 years or older (adults), 20 to <40 years (young adults), 40 to <60 years (middle-aged adults), 60 to <75 years (seniors) and 75 years or older (elderly).

In a particular preferred embodiment of the invention, the human is selected from the group consisting of infants and young children, in particular an infant, e.g., an infant having an age from 0 to 6 months. The infant may be born term or preterm. The infant may be born by C-section or vaginally.

Since viral infections, in particular infection with RSV, is associated with subsequent development of allergic airway diseases, such as asthma later in life (Feldman et al. 2015 Am J Respir Crit Care Med, 191;34-44), the composition is also effective for use in preventing or reducing the risk of allergen sensitisation and/or developing an allergic respiratory tract disease in a mammal, such as a human. Examples of allergic respiratory tract diseases include asthma and recurrent wheeze, in particular asthma.

In this case, the composition is preferably administered to a human having an age from 0 to <3 years, preferably from 0 to 2 years, more preferably from 0 to <1 year, such as from 0 to 6 months. This, in turn, prevents or reduces the risk of developing an allergic respiratory tract disease when said human has reached an age of 3 years or more, preferably from 3 to 12 years, more preferably from 3 to 10 years, even more preferably from 3 to 8, most preferably from 3 to 6 years, in particular from 3 to 5 years or from 3 to 4 years.

The product prepared by the use of the present composition may be a food product, a supplement, an animal food product or a pharmaceutical composition. For example, the product may be a nutritional composition, a nutraceutical, a drink, a food additive or a medicament.

A food additive or a medicament may be in the form of tablets, capsules, pastilles or a liquid for example. Food additives or medicaments are preferably provided as sustained release formulations, allowing a constant supply of SIgA and biotics, such as probiotics or postbiotics, for prolonged times.

The product is preferably selected from the group consisting of milk powder-based products; instant drinks; ready-to-drink formulations; nutritional powders; nutritional liquids, for example follow-on or follow-up infant formula; milk-based products, in particular yoghurts or ice cream; cereal products; beverages; water; coffee; cappuccino; malt drinks; chocolate flavoured drinks; culinary products; soups; tablets; syrups and pet food.

Milk may be any milk obtainable from animal or plant sources and is preferably cow's milk, human milk, sheep milk, goat milk, horse milk, camel milk, rice milk or soy milk.

Instead of milk, also milk derived protein fractions or colostrum may be used.

The composition may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents, gel forming agents, antioxidants and antimicrobials. They may also contain conventional pharmaceutical additives and adjuvants, excipients and diluents, including, but not limited to, water, gelatine of any origin, vegetable gums, ligninsulfonate, talc, sugars, starch, gum arabic, vegetable oils, polyalkylene glycols, flavouring agents, preservatives, stabilizers, emulsifying agents, buffers, lubricants, colorants, wetting agents, fillers, and the like. Further, they may contain an organic or inorganic carrier material suitable for oral or enteral administration as well as vitamins, minerals trace elements and other micronutrients in accordance with the recommendations of Government bodies such as the USRDA.

The composition may contain a protein source, a carbohydrate source and/or a lipid source.

Any suitable dietary protein may be used, for example animal proteins (such as milk proteins, meat proteins and egg proteins); vegetable proteins (such as soy protein, wheat protein, rice protein, and pea protein); mixtures of free amino acids; or combinations thereof. Milk proteins such as casein and whey, rice and soy proteins are particularly preferred.

If the composition includes a fat source, the fat source more preferably provides 5% to 40% of the energy of the formula; for example, 20% to 30% of the energy. DHA may be added. A suitable fat profile may be obtained using a blend of canola oil, corn oil and high-oleic acid sunflower oil.

A source of carbohydrates may more preferably provide between 40% to 80% of the energy of the composition. Any suitable carbohydrate may be used, for example sucrose, lactose, glucose, fructose, corn syrup solids, maltodextrins, and mixtures thereof.

The product prepared may be administered to humans or animals, in particular companion animals, pets or livestock. It has beneficial effects for any age group.

Preferably, the product is intended in for infants, juveniles, adults or elderly. It may however also be administered to mothers during pregnancy and lactation to support the infant immune system maturation and immune imprinting.

The composition will be effective as long as the biotic and SIgA are administered simultaneously, briefly one after the other in a maximal timeframe of less than 60 minutes, preferably less than 30 minutes, more preferred less than 15 minutes and most preferred less than 5 minutes, and/or are combined prior to administration as already present in the food product. However, it was found that the combination of the biotic and SIgA is in particular effective, if SIgA and the biotic are combined in complexes prior to administration. This has the advantage that the beneficial complexes do not need to form after consumption of the product, but that they are already present in the food product.

SIgA and at least one bacterium are present as complexes, for example in a way that at least 50%, preferably 70%, more preferably at least 90%, even more preferred all bacteria are present as a complex in association with at least 1 SIgA molecule, for example with at least 5 SIgA molecules.

The present disclosure may also relate to a pharmacological composition or food product comprising SIgA and at least one biotic. The SIgA and the biotic are combined as a complex in the food composition. SIgA and the biotics may preferably be present in a stoichiometric ratio of at least 10:1, preferably at least 100:1, most preferably at least 2000:1 to 100000:1. The upper limit of SIgA saturation is determined by the surface of the biotic, such as probiotic and postbiotic, and by the number of available binding sites for SIgA.

The composition may also contain at least one other kind of beneficial bacteria, their components and/or inanimate bacteria preparations preferably selected from the group consisting of lactic acid bacteria, bifidobacteria, enterococci, Akkermansia, Faecalibacterium or mixtures thereof. These other food grade bacteria may contribute to obtain a healthy gut microflora and will hence contribute to achieve the object of the present invention even more effectively.

Typically, the probiotics will be effective in a large range amount. If the bacteria reach the intestine alive, a single bacterium can be sufficient to achieve a powerful effect by persisting in the gut and multiplication. However, in general, it is preferred if the product comprises between 10<2> and 10<10> cells of probiotics per daily dose.

The amount of SIgA required to achieve an effect, is equally not limited. In principle one SIgA molecule combined with one probiotic micro-organism, preferably in the form of a complex, will be effective for the present invention. However, it is in generally preferred if the product comprises between 0.0001 mg SIgA and 100 mg SIgA per daily dose, more preferably between 0,0001 mg and 100 mg SIgA per daily dose.

Further advantages and features of the present invention are apparent from the following Examples.

### Examples

### Neonatal mouse model of PVM (pneumonia virus of mice)-induced bronchiolitis

To test potential benefits of early life nutritional supplementation with slgA-biotics to provide protection from viral airway infection, neonatal *pIgR*^{*+*/*-*} C57BL/6 mice reared by a *pIgR*^{*-*/*-*} dam(no passive transfer of sIgA through milk; susceptible groups) were fed once daily (i.g) with sigA-*B*. *infantis* complex (10⁵ CFU/day) in combination with a mixture of 5 HMOs (2FL, DFL, LNT, 3SL and 6SL); 2 mg total HMOs/day)

At postnatal day (PND) 5, litters were randomly attributed to the different experimental groups and *pIgR*^{*+*/*-*} pups were fed once daily with different combinations of nutritional ingredients via gastric gavage (i.g.) of 50 µl of ingredients in PBS ending PND20 (feeding lasted for 15 days) At PND10, all animals were infected intra-nasally (i.n.) with 10 PFU of PVM (J3666) in a total volume of 40 µL in a saline solution (20 µL/nostril) to induce bronchiolitis.

Neonatal *pIgR^{+l-}* C57BL/6 mice reared by a *pIgR*^{*+*/*+*} mother (passive sIgA transfer to the offspring through milk; protected group) were used as a positive control.

Mice were sacrificed either at (i) PND15 to assess peak of viral response (viral load, anti-viral immune response) or (ii) PND20 to measure magnitude of lung inflammation and associated pathology. Immune responses to infection and lung tissue remodelling were assessed.

Live sigA-Biotic ingredient was produced by complexing freshly produced *B. infantis* (NCC 3089, LMG 11588, 2x10⁶ CFU) with purified bovine SIgA (10µg, isolated from microfiltered bovine skimmed milk) for 30 min at 37°C under agitation. Complexation rate for live sIgA-biotic was assessed by flow cytometry (>90% sIgA-biotic complexes).

Postbiotic sIgA-biotic was produced by exposing live *B. infantis* to heat treatment for 10 min at 82°C. Following heat treatment of *B. infantis,* complexation of postbiotic with SIgA was performed as described for live sIgA-biotic(2x10⁶ CFU *B. infantis* coated with 10µg SigA). Complexation rate for postbiotic sIgA-biotic was assessed by flow cytometry (>90% sIgA-biotic complexes).

A mix of 5 HMO (2FL/DFL, LNT, 3SL, 6SL) was provided in some groups at a dose of 2mg/day. The ratio between the 4 ingredients was: 2FL/DFL ingredient 65.70%; LNT ingredient 23.40%; 6SL ingredient 9%, 3SL ingredient 2%. This ratio is inspired on breast milk composition.

A dose 10⁵ CFU or AFU/day of *B.Infantis* (live or heat treated) was given in groups treated with probiotic only.

### Example 1:

To assess whether early life nutritional supplementation with slgA-biotics (live or as postbiotic) provide protection from viral airway infection, viral load in PVM-infected mice was assessed as the percentage of PVM-positive airway epithelial cells (Figure 1C and Figure 4). Surprisingly, nutritional supplementation with slgA-biotics (live or as postbiotic) in presence (Figure 1C) or absence of 5HMOs (Figure4) resulted in a significant decrease in virus infected airway epithelial cells 5 days post infection (dpi) compared to susceptible control group.

### Example 2:

Plasmacytoid dendritic cells (pDCs) are critical regulators of viral infections. pDCs recognize invading viruses and rapidly produce vast amounts of type 1 and type 3 interferons. This process promotes an antiviral state, and the airway epithelium fortifies itself against the virus using different mechanisms. An appropriate immune response is generated, the virus gets cleared and regulatory T cells induced by pDCs help to dampen local inflammation and restore tissue homeostasis.

To assess whether early life nutritional supplementation with slgA-biotics (live or as postbiotic) has an impact on immune cell composition, numbers of pDCs were quantified in lung tissues of PVM-infected mice 5 days post infection using multi-colour flow cytometry. Both protected controls and susceptible animals supplemented with slgA-biotics (live or as postbiotic) in presence (Figure 5) or absence of 5HMO (Figure 1A), demonstrated with significantly increased numbers of pDCs in the airways compared to susceptible controls.

Given the known effect of pDCs on regulatory T cells (Tregs), a flow cytometric approach was used to quantify Treg (CD45⁺, CD4⁺, CD25⁺, Foxp3⁺) numbers in lung tissues of PVM-infected mice. At 5 days post infection (peak of viral infection), nutritional supplementation with slgA-biotic (live or as postbiotic) in the presence (Figure 6) or absence of 5HMOs (Figure 1B) resulted in significantly higher numbers of Tregs in lung tissues of infected mice compared to susceptible controls. Nutritional supplementations with slgA-biotic + 5HMOseven reached higher levels than protected controls.

### Example 3:

Pathological hallmarks of a severe viral lower respiratory infection include airway epithelial cell (AEC) sloughing, mucus hypersecretion and airway smooth muscle (ASM) remodelling. AEC detachemnt is a feature of viral bronchiolitis and is associated with disease severity and viral load. To investigate the impact of early life nutritional supplementation with slgA-biotics (live or as postbiotic) in the presence or absence of 5HMOs, AEC sloughing was assessed at 10 day post infection in different groups. AEC sloughing was quantified by measuring the length of sloughed airway epithelium and expressing this as a percentage as basement membrane length of airway in at least five airways per mouse. Early life supplementation with slgA-biotic (live or as postbiotic) significantly reduced AEC sloughing compared to susceptible controls (Figure 2A).

Mucus hyper-secretion and pathological lung tissue remodeling as other hallmarks of severe bronchiolitis were assessed by quantification of Muc5ac positive AECs, and ASM remodeling was assessed by area/airway circumference espectively (Lynch JP, JEM 2018)Early life nutritional supplementation with slgA-biotics (live or as postbiotic) significantly reduced PVM-induced mucus hypersecretion (Figure 2B) and pathological lung tissue remodeling (late phase) compared to susceptible controls (Figure 2C).

### Example 4:

To assess whether early life nutritional supplementation with IgA-biotics (live or as postbiotic), besides eliciting an effective anti-viral immune response, results in controlled resolution of lung inflammation upon viral clearance, numbers of neutrophils, eosinophils and CD8-positive T cells in the airways of PVM infected mice were assessed by flow cytometry at 10 days post infection (late phase). Importantly, early life supplementation with slgA-biotics (live or as postbiotic) resulted in controlled resolution of lung inflammation upon clearance of virus as assessed by significantly reduced numbers of granulocytes (neutrophils and eosinophils) and CD8 T cells in the airways in response to PVM infection (Figure 3A-C). These data indicate that early life supplementation with IgA-biotics (live or as postbiotic) control effective anti-viral immune responses and promote rapid resolution of lung inflammation and thus recovery from respiratory infection.

## Claims

1. A composition comprising a secretory immunoglobulin A-biotic (slgA-biotic) complex, wherein said biotic is a bacterium, wherein said bacterium is a *Bifidobacterium* or a *Lactobacillus,*
for use in the prevention and/or treatment of a viral infection in a mammal;
wherein for use in the prevention and/or treatment of a viral infection in a mammal is
for use in reducing the risk of contracting a viral infection in a mammal and/or for use in reducing the symptoms associated with a viral infection in a mammal, and/or
for use in preventing or reducing the risk of contracting a bacterial co-infection and/or a bacterial secondary infection associated with respiratory viral infections in a mammal,
wherein said viral infection is a viral respiratory tract infection.

2. The composition for use according to claim 1, wherein said bacterium is a *Bifidobacterium.*

3. The composition for use according to claim 2, wherein said *Bifidobacterium* is selected from the group consisting of *Bifidobacterium infantis, Bifidobacterium breve, Bifidobacterium longum, Bifidobacterium juvenis, Bifidobacterium lactis, Bifidobacterium bifidum, Bifidobacterium adolescentis* and *Bifidobacterium animalis.*

4. The composition for use according to claim 1, wherein said bacterium is a *Lactobacillus.*

5. The composition for use according to claim 4, wherein said *Lactobacillus* is selected from the group consisting of *Lactobacillus acidophilus, Lactobacillus rhamnosus, Lactobacillus casei, Lactobacillus paracasei, Lactobacillus plantarum, Lactobacillus reuteri, Lactobacillus salivarius* and *Lactobacillus johnsonii.*

6. The composition for use according to any of the preceding claims, wherein said composition is a nutritional composition.

7. The composition for use according to claim 6, wherein said nutritional composition is selected from the group consisting of an infant formula, such as a starter infant formula or a follow-on formula; a baby food; an infant cereal composition; a growing-up milk; a fortifier, such as a human milk fortifier; and a supplement.

8. The composition for use according to claim 7, wherein said nutritional composition is an infant formula.

9. A composition for use as defined in any of claims 1-8 for use in preventing or reducing the risk of allergen sensitisation and/or developing an allergic respiratory tract disease in a mammal.

10. The composition for use according to any of the preceding claims, wherein said viral infection causes a disease selected from the group consisting of common cold, influenza (flu), bronchitis, bronchiolitis and pneumonia.

11. The composition for use according to claim 10, wherein said disease is selected from the group consisting of bronchiolitis and pneumonia.

12. The composition for use according to any of the preceding claims, wherein said symptoms associated with the viral infection are selected from the group consisting of irritation in the lungs, congestion in the lungs, excessive mucus production, fever, cough, wheezing, breathlessness, abdominal cramps, diarrhoea and vomiting.

13. The composition for use according to any of the preceding claims, wherein said infection is caused by a virus selected from the group consisting of respiratory syncytial virus (RSV), parainfluenza virus (PIV), influenza virus such as influenza virus A (IVA) and/or influenza virus B (IVB), rhinovirus (RV), adenovirus (ADV), metapneumovirus (MPV), bocavirus (BoV), coronavirus (CoV), myxovirus, enterovirus (EV), parachovirus (PeV) or a combination thereof.

14. The composition for use according to claim 13, wherein said infection is caused by respiratory syncytial virus (RSV).

15. The composition for use according to claim 14, wherein the respiratory syncytial virus (RSV), is associated with subsequent development of allergic airway diseases, such as asthma later in life.

## Patentansprüche

1. Zusammensetzung, umfassend einen sekretorischen Immunglobulin-A-Biotika-Komplex (slgA-Biotika-Komplex), wobei das Biotika ein Bakterium ist, wobei das Bakterium ein *Bifidobacterium* oder ein *Laktobacillus* ist,
zur Verwendung bei einer Vorbeugung und/oder Behandlung einer Virusinfektion bei einem Säugetier;
wobei zur Verwendung bei einer Vorbeugung und/oder Behandlung einer Virusinfektion bei einem Säugetier ist
zur Verwendung bei einer Verringerung des Risikos einer Ansteckung mit einer Virusinfektion bei einem Säugetier und/oder zur Verwendung bei einer Verringerung der Symptome, die einer Virusinfektion zugeordnet sind, bei einem Säugetier und/oder
zur Verwendung bei einer Vorbeugung oder Verringerung des Risikos einer Ansteckung mit einer bakteriellen Co-Infektion und/oder einer bakteriellen Sekundärinfektion, die viralen Atemwegsinfektionen zugeordnet sind, bei einem Säugetier,
wobei die Virusinfektion um eine virale Infektion der Atemwege ist.

2. Zusammensetzung zur Verwendung nach Anspruch 1, wobei das Bakterium ein *Bifidobacterium* ist.

3. Zusammensetzung zur Verwendung nach Anspruch 2, wobei das *Bifidobacterium* aus der Gruppe ausgewählt ist, bestehend aus *Bifidobacterium infantis, Bifidobacterium breve, Bifidobacterium longum, Bifidobacterium juvenis, Bifidobacterium lactis, Bifidobacterium bifidum, Bifidobacterium adolescentis* und *Bifidobacterium animalis.*

4. Zusammensetzung zur Verwendung nach Anspruch 1, wobei das Bakterium ein *Lactobacillus* ist.

5. Zusammensetzung zur Verwendung nach Anspruch 4, wobei das *Lactobacillus* aus der Gruppe ausgewählt ist, bestehend aus *Lactobacillus acidophilus, Lactobacillus rhamnosus, Lactobacillus casei, Lactobacillus paracasei, Lactobacillus plantarum, Lactobacillus reuteri, Lactobacillus salivarius* und *Lactobacillus johnsonii.*

6. Zusammensetzung zur Verwendung nach einem der vorstehenden Ansprüche, wobei die Zusammensetzung eine Nährstoffzusammensetzung ist.

7. Zusammensetzung zur Verwendung nach Anspruch 6, wobei die Nährstoffzusammensetzung aus der Gruppe ausgewählt ist, bestehend aus einer Säuglingsanfangsnahrung, wie eine Startersäuglingsanfangsnahrung oder eine Folgenahrung; einer Babynahrung; einer Säuglingsgetreidezusammensetzung; einer Wachstumsmilch; einem Anreicherungsmittel, wie ein Humanmilchanreicherungsmittel; und einem Nahrungsergänzungsmittel.

8. Zusammensetzung zur Verwendung nach Anspruch 7, wobei die Nährstoffzusammensetzung eine Säuglingsanfangsnahrung ist.

9. Zusammensetzung zur Verwendung nach den Ansprüchen 1 bis 8 zur Verwendung bei einer Vorbeugung oder Verringerung des Risikos einer Allergen-Sensibilisierung und/oder einer Entwicklung einer allergischen Atemwegserkrankung bei einem Säugetier.

10. Zusammensetzung zur Verwendung nach einem der vorstehenden Ansprüche, wobei die Virusinfektion eine Erkrankung verursacht, die ausgewählt ist aus der Gruppe bestehend aus Erkältung, Grippe, Bronchitis, Bronchiolitis und Lungenentzündung.

11. Zusammensetzung zur Verwendung nach Anspruch 10, wobei die Erkrankung aus der Gruppe ausgewählt ist, bestehend aus Bronchiolitis und Lungenentzündung.

12. Zusammensetzung zur Verwendung nach einem der vorstehenden Ansprüche, wobei die Symptome, die der Virusinfektion zugeordnet sind, aus der Gruppe ausgewählt sind, bestehend aus Reizung in der Lunge, Stauung in der Lunge, übermäßiger Schleimproduktion, Fieber, Husten, Keuchen, Atemnot, Bauchkrämpfen, Durchfall und Erbrechen.

13. Zusammensetzung zur Verwendung nach einem der vorstehenden Ansprüche, wobei die Infektion durch ein Virus verursacht wird, das aus der Gruppe ausgewählt ist, bestehend aus Respiratorischem Synzytialvirus (RSV), Parainfluenzavirus (PIV), Influenzavirus wie Influenzavirus A (IVA) und/oder Influenzavirus B (IVB), Rhinovirus (RV), Adenovirus (ADV), Metapneumovirus (MPV), Bocavirus (BoV), Coronavirus (CoV), Myxovirus, Enterovirus (EV), Parachovirus (PeV) oder einer Kombination davon.

14. Zusammensetzung zur Verwendung nach Anspruch 13, wobei die Infektion durch das Respiratorische Synzytialvirus (RSV) verursacht wird.

15. Zusammensetzung zur Verwendung nach Anspruch 14, wobei das Respiratorische Synzytialvirus (RSV) einer nachfolgenden Entwicklung allergischer Atemwegserkrankungen, wie Asthma im späteren Lebensalter, zugeordnet ist.

## Revendications

1. Composition comprenant un complexe immunoglobuline A sécrétoire-biotique (slgA-biotique), dans laquelle ledit biotique est une bactérie, dans laquelle ladite bactérie est un *Bifidobacterium* ou un *Lactobacillus,*
pour une utilisation dans la prévention et/ou le traitement d'une infection virale chez un mammifère ;
dans laquelle pour une utilisation dans la prévention et/ou le traitement d'une infection virale chez un mammifère est
pour une utilisation dans la réduction du risque de contracter une infection virale chez un mammifère et/ou pour une utilisation dans la réduction des symptômes associés à une infection virale chez un mammifère, et/ou
pour une utilisation dans la prévention ou la réduction du risque de contracter une co-infection bactérienne et/ou une infection secondaire bactérienne associée à des infections virales respiratoires chez un mammifère,
dans laquelle ladite infection virale est une infection virale des voies respiratoires.

2. Composition pour une utilisation selon la revendication 1, dans laquelle ladite bactérie est un *Bifidobacterium.*

3. Composition pour une utilisation selon la revendication 2, dans laquelle ledit *Bifidobacterium* est choisi dans le groupe constitué de *Bifidobacterium infantis, Bifidobacterium breve, Bifidobacterium longum, Bifidobacterium juvenis, Bifidobacterium lactis, Bifidobacterium bifidum, Bifidobacterium adolescentis* et *Bifidobacterium animalis.*

4. Composition pour une utilisation selon la revendication 1, dans laquelle ladite bactérie est un *Lactobacillus.*

5. Composition pour une utilisation selon la revendication 4, dans laquelle ledit *Lactobacillus* est choisi dans le groupe constitué de *Lactobacillus acidophilus, Lactobacillus rhamnosus, Lactobacillus casei, Lactobacillus paracasei, Lactobacillus plantarum, Lactobacillus reuteri, Lactobacillus salivarius* et *Lactobacillus johnsonii.*

6. Composition pour une utilisation selon l'une quelconque des revendications précédentes, dans laquelle ladite composition est une composition nutritionnelle.

7. Composition pour une utilisation selon la revendication 6, dans laquelle ladite composition nutritionnelle est choisie dans le groupe constitué d'une préparation pour nourrissons, telle qu'une préparation pour nourrissons du premier âge ou une préparation de suite ; un aliment pour bébés ; une composition de céréales pour nourrissons ; un lait de croissance ; un fortifiant, tel qu'un fortifiant du lait maternel ; et un complément.

8. Composition pour une utilisation selon la revendication 7, dans laquelle ladite composition nutritionnelle est une préparation pour nourrissons.

9. Composition pour une utilisation selon l'une quelconque des revendications 1 à 8 pour une utilisation dans la prévention ou la réduction du risque de sensibilisation à un allergène et/ou de développement d'une maladie allergique des voies respiratoires chez un mammifère.

10. Composition pour une utilisation selon l'une quelconque des revendications précédentes, dans laquelle ladite infection virale provoque une maladie choisie dans le groupe constitué du rhume, de la grippe, de la bronchite, de la bronchiolite et de la pneumonie.

11. Composition pour une utilisation selon la revendication 10, dans laquelle ladite maladie est choisie dans le groupe constitué de bronchiolite et de pneumonie.

12. Composition pour une utilisation selon l'une quelconque des revendications précédentes, dans laquelle lesdits symptômes associés à l'infection virale sont choisis dans le groupe constitué d'irritation dans les poumons, congestion dans les poumons, production excessive de mucus, fièvre, toux, respiration sifflante, essoufflement, crampes abdominales, diarrhée et vomissements.

13. Composition pour une utilisation selon l'une quelconque des revendications précédentes, dans laquelle ladite infection est causée par un virus choisi dans le groupe constitué du virus respiratoire syncytial (VRS), virus parainfluenza (PIV), virus de la grippe tel que le virus de la grippe A (IVA) et/ou le virus de la grippe B (IVB), rhinovirus (RV), adénovirus (ADV), métapneumovirus (MPV), bocavirus (BoV), coronavirus (CoV), myxovirus, entérovirus (EV), parachovirus (PeV) ou une combinaison de ceux-ci.

14. Composition pour une utilisation selon la revendication 13, dans laquelle ladite infection est causée par le virus respiratoire syncytial (VRS).

15. Composition pour une utilisation selon la revendication 14, dans laquelle le virus respiratoire syncytial (VRS) est associé au développement ultérieur de maladies allergiques des voies respiratoires, telles que l'asthme, à un stade ultérieur de la vie.
